# EUROPEAN PATENT SPECIFICATION

(11) **EP 3 442 618 B1**
(45) Date of publication and mention of the grant of the patent: **17.03.2021**
(21) Application number: 17783288.8
(22) Date of filing: 14.04.2017
(51) Int. Cl.: B04B 5/04, B04B 7/06, A61M 1/36, A61M 1/38, A61M 1/34, B04B 11/04, B04B 13/00, G06K 9/00, G06K 9/03, G06K 9/62

(54) **LOADING OF DISPOSABLE**
LADEN VON EINWEGARTIKEL
CHARGEMENT D'ARTICLE JETABLE

(30) Priority: 14.04.2016 US 201662322723 P
(43) Date of publication of application: 20.02.2019
(73) Proprietor: Terumo BCT, Inc., Lakewood, CO 80215 (US)
(72) Inventor: STANTON, Briden Ray, Highlands Ranch Colorado 80129 (US); BAEVERSTAD, Brett Michael, Fort Collins Colorado 80528 (US); SCIBONA, Joseph A., Littleton Colorado 80128 (US); NGUYEN, Minh Anh Thi, Fort Collins Colorado 80525 (US); RIVES, Megan A., Timnath, CO 80547 (US)
(74) Representative: J A Kemp LLP
(86) International application number: PCT/US2017/027723
(87) International publication number: WO 2017/181077

(56) References cited:
- WO-A1-2016/008755
- JP-A- 2000 353 684
- US-A1- 2004 245 189
- US-A1- 2005 084 961
- US-A1- 2007 093 774
- US-A1- 2007 243 990
- US-A1- 2013 148 883
- US-A1- 2014 045 668

## Description

### Background

There are two common methods for blood donation/collection. The first is whole blood donation from a donor, followed by centrifugal processes to separate blood components by density. The desired component may then be manually or semi-automatically pressed into a different sterile collection bag for further use. The other method may be referred to as apheresis collection and it is achieved using a specialized machine.

The apheresis method may to used to collect only a blood component that is desired and has the ability to route all other blood components back into a donor within the same procedure. Apheresis machines are also designed for the transfer of specific blood components to patients that are in need. The success of each individual procedure is reliant upon the operator's ability to correctly load a disposable kit into the machine by engaging portions of the disposable with portions of the machine. There may be a multiple step set-up and configuration for every procedure performed.

Apheresis systems may utilize a disposable that is engaged with/positioned in the apheresis machine in a predetermined way. If the kits are not positioned correctly, there may be leaks. The leaks may cause a loss of blood components and terminate a patient's procedure prior to completion.

Embodiments of the present invention have been made in light of these and other considerations. However, the relatively specific problems discussed above do not limit the applicability of the embodiments of the present invention.

US 2007/0243990 discloses a system and a method for identifying incorrect loading of a disposable in a fluid separation system, using ultrasonic sensors and a processor connected thereto. WO 2016/008755 and JP 2000-353684 disclose various arrangements of centrifuges and control systems therefor.

### Summary

The invention is defined in the claims. The summary is provided to introduce aspects of some embodiments of the present invention in a simplified form, and is not intended to identify key or essential elements of the claimed invention, nor is it intended to limit the scope of the claims.

Embodiments may provide for methods and devices that detect the position of disposables after mounting in medical devices to determine if portions of the disposable are positioned in desired, predetermined ways. Embodiments may involve optical systems, audio system, and combinations thereof to detect possible incorrect loading of disposables. In some embodiments, the medical devices may be blood separation machines, such as apheresis machines.

### Brief Description of the Drawings

Non-limiting and non-exhaustive embodiments are described with reference to the following figures.
FIG. 1 illustrates one embodiment of an apheresis system, which can be used in, or with, embodiments.
FIG. 2 illustrates a tubing and bag set for use in, or with, embodiments.
FIG. 3 illustrates a centrifuge assembly and a system for detecting a position of a disposable according to embodiments.
FIGS. 4A and 4B are two images showing a light source and scattering of light according to an embodiment.
FIG. 5A is an image showing a first position where a disposable may be engageable with a centrifuge assembly with the disposable engaged incorrectly.
FIG. 5B is an image showing a second position where a disposable may be engageable with a centrifuge assembly with the disposable engaged incorrectly.
FIG. 6A is an image of a position where a disposable may be engageable with a centrifuge assembly showing the disposable engaged correctly.
FIG. 6B is an image of a position where a disposable may be engageable with a centrifuge assembly showing the disposable engaged incorrectly.
FIG. 7A is an image of a portion of the centrifuge assembly after loading of a disposable correctly.
FIG. 7B is an image of a portion of the centrifuge after loading of a disposable incorrectly.
FIG. 8 illustrates a flow chart of a process for identifying an incorrectly loaded disposable according to an embodiment.
FIG. 9 illustrates a flow chart of a process for identifying an incorrectly loaded disposable according to another embodiment.
FIG. 10 illustrates a flow chart of a process for identifying an incorrectly loaded disposable according to yet another embodiment.
FIG. 11 illustrates a computer system that may be used to implement embodiments.

### Detailed Description

The principles of the present invention may be further understood by reference to the following detailed description and the embodiments depicted in the accompanying drawings. It should be understood that although specific features are shown and described below with respect to detailed embodiments, the present invention is not limited to the embodiments described below.

Embodiments below are described with respect to an apheresis machine used to separate whole blood into blood components. However, this is done simply for illustrative purposes. It is noted that the embodiments are not limited to the description below. The embodiments are intended for use in products, processes, devices, and systems that involve loading and positioning components, such as disposables in a system. Accordingly, the present invention is not limited to separation of whole blood into blood components.

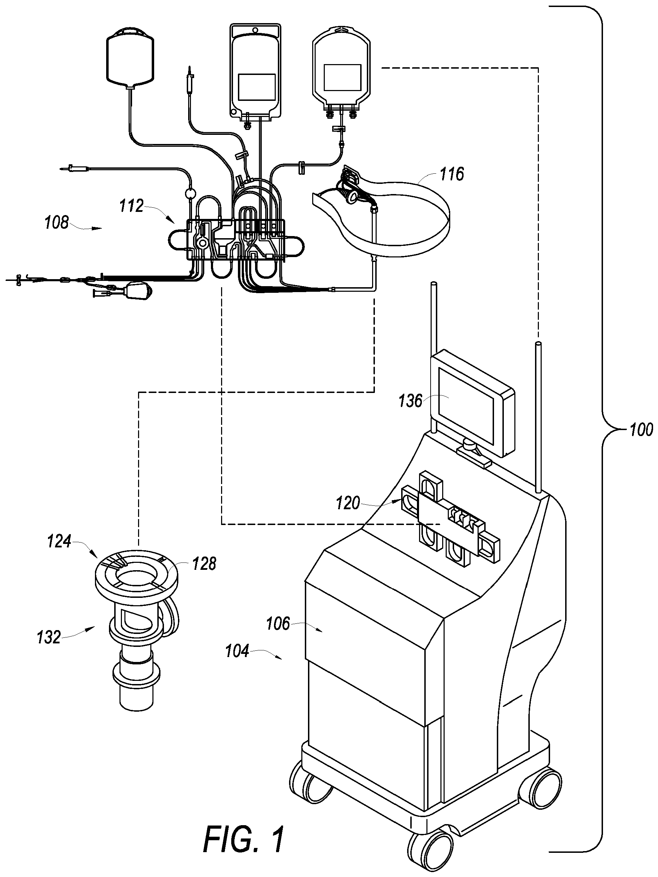

FIG. 1 illustrates one embodiment of an apheresis system 100, which can be used in, or with, embodiments. In embodiments, apheresis system 100 provides for a continuous whole blood separation process. In one embodiment, whole blood is withdrawn from a donor and is substantially continuously provided to a blood component separation device 104 where the blood is separated into various components and at least one of these blood components is collected from the device 104. One or more of the separated blood components may be either collected for subsequent use or returned to the donor. In embodiments, blood is withdrawn from the donor and directed through a bag and tubing set 108, which includes an extracorporeal tubing circuit 112, and a blood processing vessel 116, which together define a closed, sterile and disposable system. The set 108 is adapted to be mounted in the blood component separation device 104. The separation device 104 includes a pump/valve/sensor assembly 120, which interfaces with the extracorporeal tubing circuit 112, and a centrifuge assembly 124, which interfaces with the blood processing vessel 116 and other portions of set 108.

Examples of apheresis and other separation systems that may be used with embodiments of the present invention, e.g., as system 100, include the SPECTRA OPTIA® apheresis system, COBE® spectra apheresis system, and the TRIMA ACCEL® automated blood collection system, all manufactured by Terumo BCT, of Lakewood, Colorado.

The centrifuge assembly 124 may include a channel 128 in a rotatable rotor assembly 132, which provides the centrifugal forces that may separate blood into its various blood component types by centrifugation. The blood processing vessel 116 may then be fitted within the channel 128. Blood can flow substantially continuously from the donor, through the extracorporeal tubing circuit 112, and into the rotating blood processing vessel 116. Within the blood processing vessel 116, blood may be separated into various blood component types and at least one of these blood component types (e.g., white blood cells, platelets, plasma, or red blood cells) may be removed from the blood processing vessel 116. Blood components that are not being retained for collection or for therapeutic treatment are also removed from the blood processing vessel 116 and returned to the donor via the extracorporeal tubing circuit 112. Various alternative apheresis systems (not shown) may also make use of embodiments of the present invention, including batch processing systems (non-continuous inflow of whole blood and/or non-continuous outflow of separated blood components) or smaller scale batch or continuous RBC/plasma separation systems, whether or not blood components may be returned to the donor.

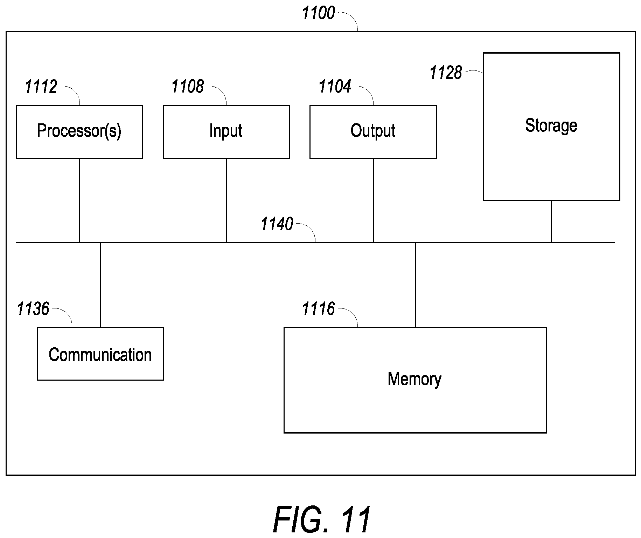

Operation of the blood component separation device 104 may be controlled by one or more processors included therein, and may advantageously comprise a plurality of embedded computer processors that are part of a computer system. The computer system may also include components that allow a user to interface with the computer system, including for example, memory and storage devices (RAM, ROM (e.g., CD-ROM, DVD), magnetic drives, optical drives, flash memory,); communication/networking devices (e.g., wired such as modems/network cards, or wireless such as Wi-Fi); input devices such keyboard(s), touch screen(s), camera(s), and/or microphone(s); and output device(s) such as display(s), and audio system(s). An example of a computer system is shown in FIG. 11 and discussed below. In order to assist the operator of the apheresis system 100 with various aspects of its operation, the embodiment of the blood component separation device 104 (shown in FIG. 1) includes a graphical user interface 136 with a display that includes an interactive touch screen.

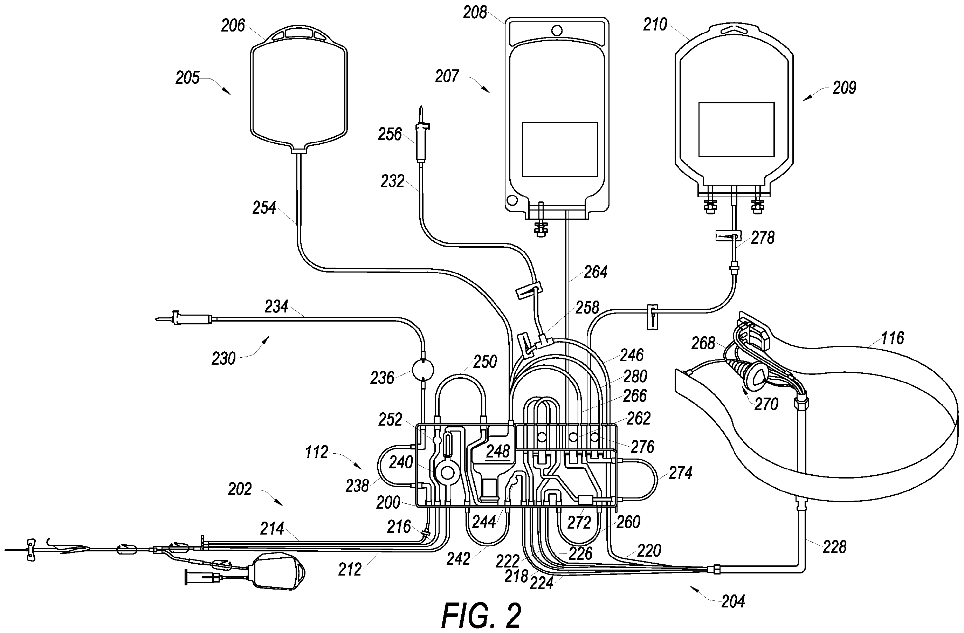

An embodiment of an extracorporeal tubing circuit is shown in FIG. 2, and as shown may include a cassette 200 and a number of tubing/collection assemblies 202, 204, 205, 207, and 209. A blood removal-return tubing assembly 202 provides a needle interface for withdrawing blood from a donor to the remainder of the tubing circuit 112 and for returning blood components and other fluids to the donor. A single needle configuration is shown, but other configuration such as a double needle interface may be used in other embodiments. Three lines 212, 214, 216 are provided in blood removal-return tubing assembly 202 for removal of blood from the donor. A cassette 200 is connected between the tubing assembly 202, which connects to the donor, and blood inlet/blood component tubing line sub-assembly 204, which provides the interface between cassette 200 and blood processing vessel 116. The cassette 200 orients tubing segments in predetermined spaced relationships within the cassette 200 for ultimate engagement with valve members on apheresis device 104. Such valves will, when activated, control flow through loops and tubing.

The tubing line sub-assembly 204 comprises five lines 218, 220, 222, 224, and 226, shown in FIG. 2, for transport of blood and components to and from the processing vessel 116. The five lines may be encased in a sheath 228, which when bundled by the sheath 228, may be described as a loop, that allows the one omega-two omega motion described in U.S. Patent No. 4,425,112. An anticoagulant tubing assembly 230, a vent bag 206, a plasma collection bag 208, and a white blood cell collection bag 210 are also interconnected with cassette 200. Optionally, a red blood cell collection assembly might also be provided through an auxiliary line 232, as is known in the art. The extracorporeal tubing circuit 112 and blood processing vessel 116 may be pre-connected to form a closed, sterilized, disposable assembly for a single use.

When the tubing circuit 112 has been mounted on the blood component separation device 104, saline solution (not shown) may prime the tubing circuit through a line 234 and filter 236 (see FIG. 2). Saline may flow through an internal passageway in the cassette 200 and through the line 214 to the distal end of the blood removal-return assembly 202. Saline can then flow up a blood withdrawal line 212 into the other tubes and passageways of the circuit 112 in preparation for blood processing. A supply or bag (not shown) of anticoagulant can then be connected to a distal end of the anticoagulant tubing assembly 230 in place of a saline supply. Anticoagulant solution may flow past the filter 236 and a first pump loop 238 through the anticoagulant line 214 to the distal end of the blood removal assembly 202. The pump loop 238 and other pump loops described herein may couple with peristaltic pumps on the blood processing device 104. A computer system and/or a processor in device 104 may control the direction and rate of flow of the fluids described herein by controlling the speed and direction of the peristaltic pumps and the position of various valves.

The blood removal line 212 may conduct blood into the cassette 200, where the blood may pass a first pressure sensor 240 and a second pump loop 242. A second pressure sensor 244, between second pump loop 242 with its associated pump and blood inflow line 218 to the blood processing vessel 116, may sense the fluid pressure effective at an inlet to the blood processing vessel 116. Emanating from blood processing vessel 116 is an RBC outlet tubing line 220 of the blood inlet/blood component tubing assembly 204. The outlet tubing line 220 may connect to an external loop 246 to a return reservoir 248. The return reservoir 248 may contact sensors on the device 104 that detect low and high fluid levels. The device 104 may be configured to keep the fluid in the reservoir 248 between these two levels by controlling flow out of the reservoir past a return pump loop 250 and a return pressure sensor 252. As the fluid level in the reservoir 248 is constantly rising and falling, a vent bag 206 may be connected to the reservoir 248 through a vent tube 254. Air can flow between the reservoir 248 and the vent bag 206 in a sterile manner. Fluid may flow into a return tube 216 in the blood removal-return assembly 202. The blood removal-return assembly 202 also comprises the line 214 for priming or anti-coagulant as described above. If desired, red blood cells could be withdrawn through auxiliary line 232 and collected in a collection bag (not shown). Alternatively, a bag containing replacement fluid (not shown) may be connected to a spike or Luer connector 256 on the replacement line 232, allowing replacement fluid to pass through the return loop 246 into the reservoir 248. Blood components and replacement fluid may then be returned to the donor. In the present embodiment, replacement line 232 is connected to return loop 246 through a junction 258 and manual closures or clamps may be provided to direct the flow of red blood cells and replacement fluid.

Plasma may also be collected from the blood processing vessel 116 into plasma collection assembly 208. When desired, plasma is withdrawn from the blood processing vessel 116 through plasma line 226 to a pump loop 260. A valve 262 diverts the plasma either into a collect tube 264 to the plasma bag 208, or into connecting loop or line 266 to the reservoir 248. Excess plasma in the reservoir 248 may be returned to the donor in the same way as red blood cells, as described above.

White blood cells and platelets may flow out of the blood processing vessel 116 through a cell line 268 into a cell separation chamber 270. The contents of the separation chamber 270 may flow out of the separation chamber through an outlet. In the cassette 200, the fluid from the separation chamber 270 may pass a red-green photo sensor 272, which may be used to control periodic flushing of white blood cells out of the cell separation chamber 270 into the collect bag 210. The selected cells may flow through a pump loop or common line 274, which engages a peristaltic pump on the separation device 104. The pump loop 274 may connect to a valved passageway in the cassette 200. The blood processing device 104 can control a valve 276 to direct white blood cells or other selected cells either into a collect tube 278 and thence into the collect bag 210, or into a connection loop or line 280 and thence into the reservoir 248. For platelet collection, excess white blood cells in the reservoir 248 may be returned to the donor in the same way as red blood cells and plasma, as described above. Alternatively, for mesenchymal stem cell (MNC) collection, wherein platelets are usually returned to the donor, the MNC may be withdrawn into the collect tube 278 for storage in the collect bag 210.

During blood removal, whole blood may be passed from a donor into tubing line 212 of blood removal tubing assembly 202. The blood is pumped by the device 104 via pump loop 242, to the blood processing vessel 116 via the cassette 200 and line 218 of the blood inlet/blood component tubing assembly 204. Separation processing may then occur on a substantially continuous basis in the blood processing vessel 116, i.e., blood flows substantially continuously therein, may be continuously separated and flow as separated components. After separation processing in vessel 116, uncollected blood components may be transferred from the processing vessel 116 to and through cassette 200 and into reservoir 248 of cassette 200, which may be filled up to a predetermined level. The blood component separation device 104 may initiate a blood return sub mode wherein components may be returned to the donor through return line 216. The cycle between blood removal and blood return sub modes may continue until a predetermined amount of blood components have been harvested. In an alternative embodiment, a double needle scheme may be used so that blood may be removed from the donor and returned to a donor through two separate needles. See, for example, U.S. Patent Application Publication No. 2010/0160137, which illustrates a double needle disposable set.

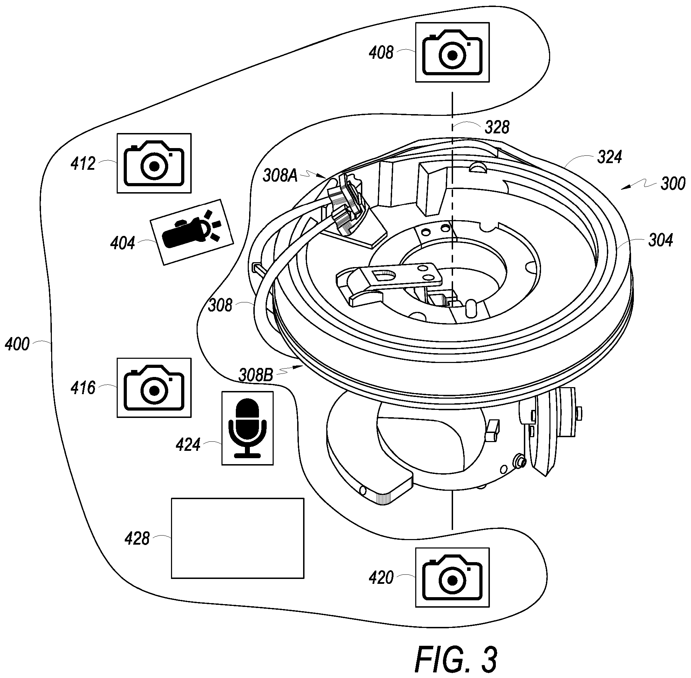

FIG. 3 illustrates a centrifuge assembly 300 and a system 400 for detecting a position of a disposable according to embodiments. Centrifuge assembly 300 may in embodiments be part of a medical device. For example, the centrifuge assembly may be part of a blood separation device, such as apheresis system 100. In these embodiments, the centrifuge assembly may be used as centrifuge assembly 124 described above.

The centrifuge assembly 300 may include a centrifuge 324, a channel 304 (in the centrifuge 324), and an arm 308. An extracorporeal tubing circuit (e.g., FIG. 2) implemented as a disposable may be mounted/positioned in centrifuge assembly 300. For example, a blood processing vessel from the disposable may be positioned within channel 304. In addition, an arm 308 of assembly 300 may include features 308A and 308B (e.g., hooks, bearings, forks, clamps, etc.) for engaging tubing from the disposable, e.g., a loop of a tubing bundle.

In embodiments, blood can flow substantially continuously from a donor, through the disposable, and into the blood vessel in channel 304. When the centrifuge 324 and the blood vessel in channel 304 are rotated around axis of rotation 328, blood may be separated into various blood component types and at least one of these blood component types (e.g., white blood cells, platelets, plasma, or red blood cells) may be removed from the blood processing vessel and collected as described above with respect to FIGS. 1 and 2.

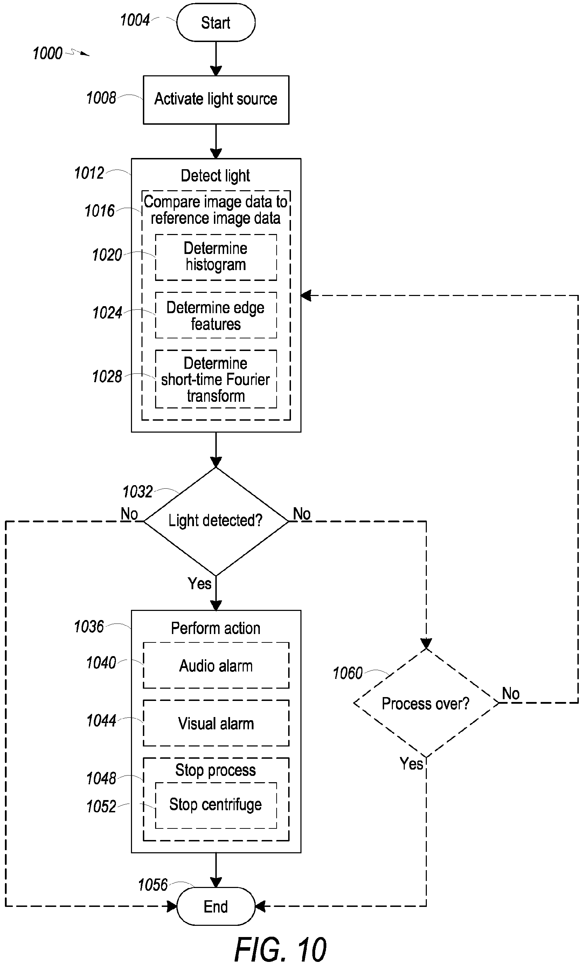

System 400 illustrates one embodiment of a system that may be used to determine whether a disposable (or portions of the disposable) have been correctly loaded (e.g., positioned) in centrifuge assembly 300. In the embodiment shown in FIG. 3, system 400 includes a light source 404, a first camera 408, a second camera 412, a third camera 416, a fourth camera 420, a microphone 424, and a processor 428, which may be part of a computer system (e.g., FIG. 10).

The light source 404 may be directed at an edge of centrifuge assembly 300. In embodiments, the light source 404 works with first camera 408 to determine whether a blood processing vessel has been properly positioned within channel 304.

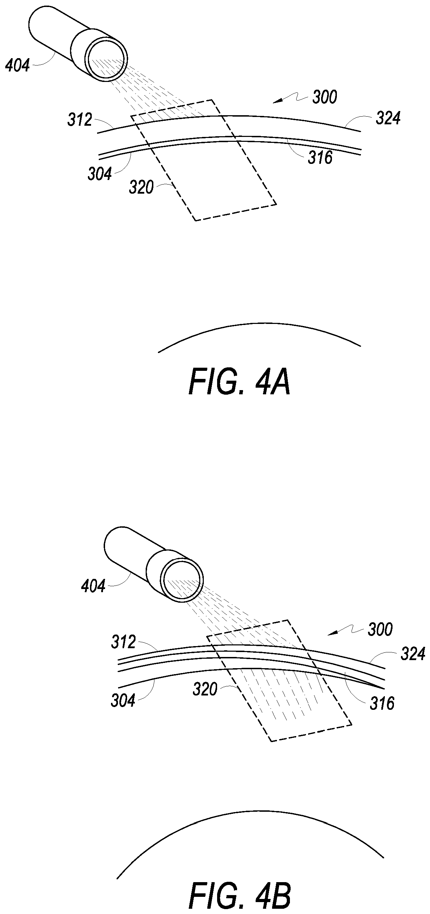

Referring to FIGS. 4A and 4B, images are shown that illustrate how a light source (e.g., light source 404) may be used to determine whether a blood processing vessel has been properly positioned within a channel of a centrifuge assembly (e.g., channel 304) according to embodiments. FIG. 4A illustrates a light source 404 that directs light at an edge 312 of centrifuge 324. In FIG. 4A, a blood processing vessel 316 is positioned correctly within channel 304. As a result of the correct positioning, there is little, if any light from source 404 in the location of assembly 300 identified by box 320.

FIG. 4B also illustrates a light source 404 directed at an edge 312 of centrifuge assembly 300. Edge 312 is adjacent channel 304. In FIG. 4B, the blood processing vessel 316 is positioned incorrectly within channel 304. As shown in FIG. 4B, at least a portion of the vessel 316 is not located within channel 304 (e.g., may protrude above channel 304). As a result of the incorrect positioning, there is light from source 404 in the location of assembly 300 identified by box 320. Without being bound by theory, it is believed that a portion of vessel 316, e.g., the portion not within channel 312, scatters light from source 404 which shines in location 320 on a top surface of centrifuge 324. In embodiments, a detector (e.g., a photodetector) may be positioned to detect light within location 320. In some embodiments, the detector may be a camera, such as camera 408 (FIG. 3).

In embodiments, light source 404 may be positioned so that light is directed to particular locations. For example, in embodiments, light source 404 may be positioned substantially parallel to a top surface of the centrifuge 324. In other embodiments, light source 404 may be positioned at a slight upward angle to a top surface of the centrifuge 324. In either position, light would not be in area 320 unless scattered by an incorrectly loaded vessel 316.

In some embodiments, if light is detected, a signal may be sent to processor 428 to perform some action. For example, processor 428 may notify an operator (e.g., audio alert, visual alert, or a combination of alerts) that a portion of a disposable has been loaded incorrectly or has moved out of a correct position. In addition to (or in lieu of) an alert, processor 428 may stop any further processing until the vessel is aligned correctly (e.g., stop rotation of centrifuge 324).

In embodiments, the second camera 412 and the third camera 416 may be used to capture image data of positions where the disposable is engageable with a portion of the centrifuge assembly 300. For example, in FIG. 3, second camera 412 is positioned to capture image data of feature 308A. Similarly, third camera 416 is positioned to capture image data of feature 308B.

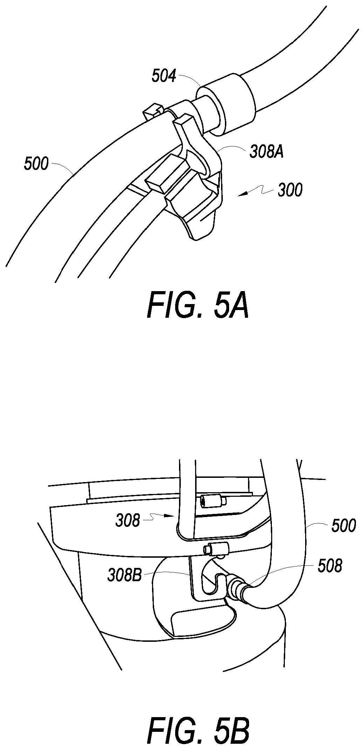

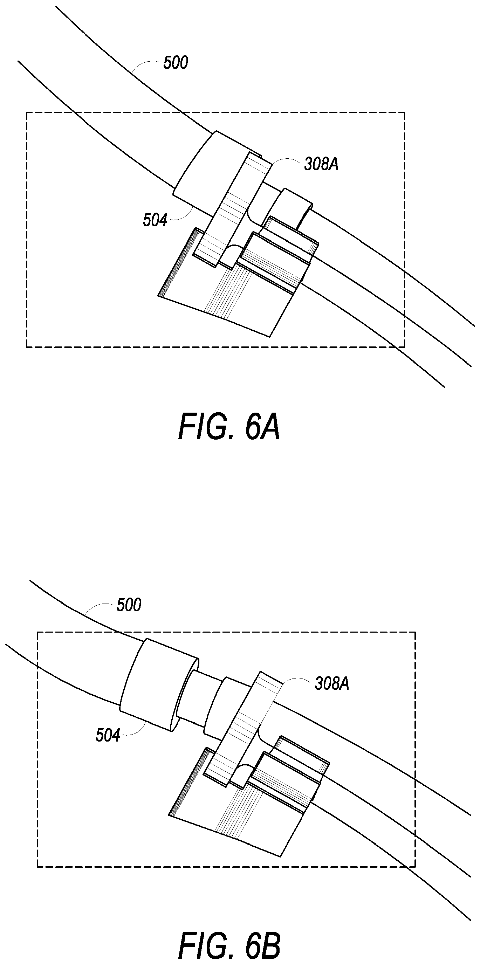

Referring now to FIGS. 5A and 5B, images are shown that illustrate additional embodiments of how portions of a disposable, e.g., a tubing loop (see e.g., FIGS. 1 and 2), may be positioned incorrectly within features of a centrifuge assembly. FIG. 5A illustrates feature 308A of arm 308. Feature 308A may be a bearing that is engageable with tubing loop 500 of a disposable. In FIG. 5A, tubing loop 500 is incorrectly positioned within bearing 308A. The correct position is to have bearing 504 of tubing loop 500 within bearing 308A of arm 308 (see e.g., FIG. 6A).

FIG. 5B illustrates feature 308B of arm 308. Feature 308B may also be a bearing that is engageable with tubing loop 500 of the disposable. In FIG. 5B, tubing loop 500 is incorrectly positioned within bearing 308B. The correct position is to have bearing 508 of tubing loop 500 within a channel of bearing 308B in arm 308.

In order to determine that a portion of a disposable is incorrectly positioned, second camera 412 and third camera 416 may capture image data at a point in time (e.g., in real time). The image data may then be compared to reference image data (e.g., taken when a disposable is correctly positioned). FIGS. 6A and 6B illustrate an example of this.

FIG. 6A shows an example of reference image data, e.g., of tubing loop 500 of a disposable engaged correctly with feature 308A. To determine if tubing loop 500 is engaged correctly at a point in time, camera 412 may capture image data, e.g., data shown in FIG. 6B. The image data captured by camera 412 may then be compared to the reference image data to determine if the tubing loop 500 is positioned correctly. FIG. 6B shows image data where tubing loop 500 of a disposable engaged incorrectly with feature 308A.

If a determination is made that the disposable (e.g., tubing loop 500) is incorrectly positioned, processor 428 may perform some action. For example, processor 428 may notify an operator (e.g., audio alert, visual alert, or a combination of alerts) that a portion of a disposable has been loaded incorrectly or has moved out of a correct position. In addition to (or in lieu of) an alert, processor 428 may stop any further processing until tubing loop 500 is positioned correctly.

In embodiments, the image data comparison may be performed by processor 428. However, in other embodiments, a camera, e.g., second camera 412 and/or third camera 416, may have a dedicated processor (or logic) that performs the comparison.

Similar to second camera 412 and third camera 416, fourth camera 420 may capture image data. In embodiments, fourth camera 420 may capture image data of a portion of a centrifuge assembly to determine whether the centrifuge assembly is operating correctly after a portion of a disposable has been loaded.

In some embodiments, in order to aid in image data comparison, features may be modified, e.g., highlighted. For example, in some embodiments, 308A and/or 308B may include highlighting features (e.g., colors, shapes, flags, etc.) that may aid in comparing image data. In one embodiment, the features 308A and/or 308B may include a color (e.g., dye or paint) that is not visible/detectable when a tubing loop is positioned correctly but visible/detectable when the tubing loop is positioned incorrectly. This is merely one example and other embodiments may provide for different forms of highlighting.

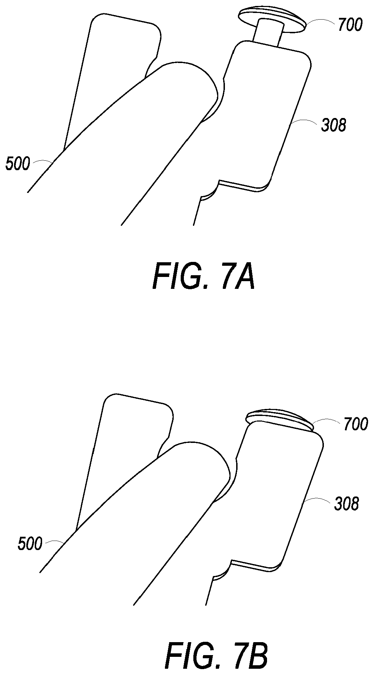

Referring to FIGS. 7A and 7B, images are shown that illustrate a pin 700 of a centrifuge assembly after a portion of a disposable, e.g., a tubing loop, is positioned in the centrifuge assembly. FIG. 7A illustrates pin 700 projected out (e.g., popped out), which is the correct position of pin 700 after a portion of the disposable, e.g., tubing loop 500, has been loaded. In embodiments, pin 700 may be part of a mechanism that holds tubing 500 in the centrifuge assembly after loading. Pin 700 may be projected out when the mechanism is working properly.

FIG. 7B illustrates pin 700 when it is not projected out. Pin 700 is not in the correct position, which may indicate that some mechanism of a centrifuge assembly may not be working properly. Similar to the description above, (e.g., description of FIGS. 6A and 6B) image data captured by camera 420 may be compared to reference image data to determine whether mechanisms (e.g., pin 700) in centrifuge assembly may be working properly.

In some embodiments, in order to aid in image data comparison, features of a centrifuge assembly may be modified, e.g., highlighted. For example, in some embodiments, pin 700 may include highlighting features (e.g., colors, shapes, flags, etc.) that may aid in comparing image data.

As noted above, in embodiments, the image data captured by camera 420 may be compared by processor 428. However, in other embodiments, camera 420 may have a dedicated processor (or logic) that performs the comparison.

In embodiments, cameras (408, 412, 416, and 420) may have features particular to application in a separation system. For example, in separation systems that may use a rotating centrifuge, the cameras may have vibration dampening features. Also, in embodiments, the cameras may have shutter speeds that allow image data to be taken of features that are on a rotating centrifuge. These are merely some examples, and cameras used in embodiments may have additional features.

System 400 also includes a microphone 424. In embodiments, microphone 424 is used to record audio data. Similar to the description of image data above, the audio data recorded by microphone 424 is compared to reference audio data to determine whether a portion of a disposable is positioned correctly in a centrifuge assembly.

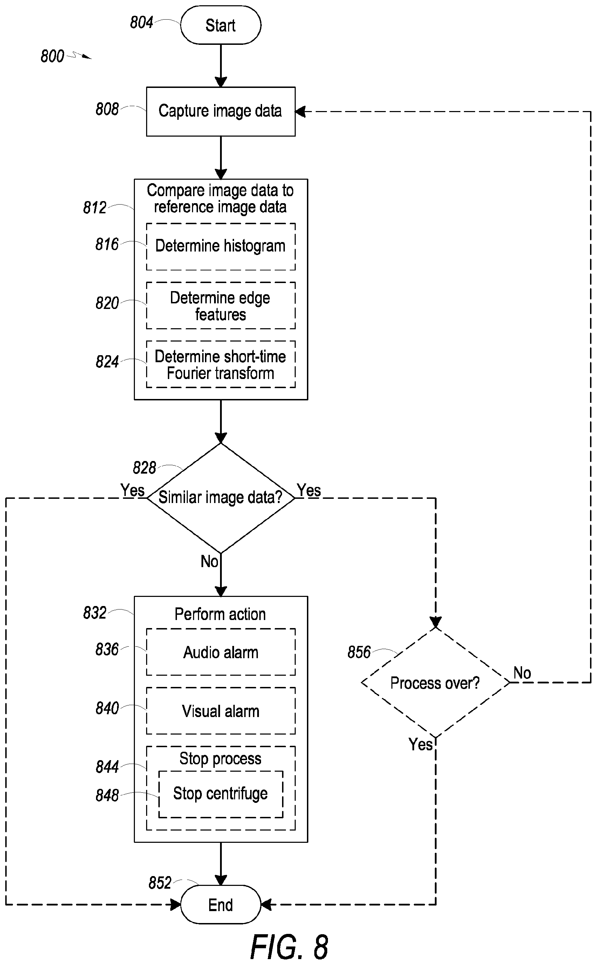

FIGS. 8, 9, and 10 illustrate flow charts 800, 900, and 1000respectively of processes for determining that a component in a system has been loaded incorrectly. In one embodiment, flow charts 800, 900, and 1000 are used to identify that a disposable component has been loaded incorrectly into a separation system (e.g., FIG. 1). Although features of a separation system (e.g., a centrifuge assembly, centrifuge, disposable, one or more processors, etc.) that may be used to separate a composite liquid (e.g., whole blood) into components may be described as part of performance of the steps of the flow chart 800, 900, and 1000 embodiments are not limited thereto. Indeed, other types of devices that may utilize components, such as disposables loaded into a system, may implement aspects of some embodiments of the present invention. The following description therefore does not limit embodiments to blood separation systems and/or methods.

Prior to performing flows 800, 900, and 1000 a number of steps may be performed in some embodiments. For example, as noted above an operator may load a disposable fluid circuit into a centrifuge assembly, prior to a process of separating whole blood into components. In embodiments, flows 800, 900, and 1000 may be performed to determine whether any portion of the disposable fluid circuit has been loaded incorrectly prior to the separation process.

Referring to FIG. 8, flow chart 800 starts at 804, and passes to step 808 where image data is captured. The image data may be of a first position where a disposable is engageable with a portion of the separation system, e.g., a centrifuge assembly. As one example, the image may be of a location where a bearing of a centrifuge assembly is engageable with a tubing loop of a disposable (e.g., FIGS. 5A-6B).

In embodiments, the image data may be captured by one or more cameras. In some embodiments, the one or more cameras that may be involved in performing step 808 may also be utilized for different functions. For example, in some embodiments, the camera utilized to capture image data at step 808, may also be used to determine an interface between components being separated. The interface location may aid in determining how fast to have fluid enter a centrifuge assembly where the fluid is being separated. In other words, step 808 may be performed by portions of an optical system designed to perform a number of functions within a separation system.

From step 808, flow 800 passes to step 812, where the image data captured at step 808 is compared to reference image data. The reference image data may have been previously captured and stored for comparison during performance of flow 800. In embodiments, step 812 may be performed, at least in part, by one or more processors. The at least one processor may in embodiments be part of a computer system such as system 1100 in FIG. 11 described below.

In some embodiments, the at least one processor may be a central processor that performs a number of other functions. In other embodiments, the at least one processor may be associated with a camera and be located near the location of the camera. Accordingly, the at least one processor may in embodiments be configured to specifically perform step 812, any of the optional steps 816, 820, and 824, as well as determination 828 (described further below).

As shown in FIG. 8, step 812 may involve a number of sub-steps. For example, the comparison may involve determining a histogram of the image data captured at optional step 816. The histogram determined at optional step 816 may be compared with a histogram of first reference data in performing step 812.

Similarly, step 812 may involve determining edge features at optional step 820 of the image data captured at step 808. The edge features of the image data captured at step 808 may be compared with edge features of reference data. In yet another embodiment, optional step 824 may be performed to determine a short-time Fourier transform of the image data captured at step 808. The short-time Fourier transform of the image data captured at step 808 may be compared with short-time Fourier transform of reference data.

In other embodiments, more than one of the optional steps 816, 820, and/or 824 may be performed during step 812. For example, in some embodiments, a combination of algorithms/methods may be used to make more accurate comparisons of the image data captured at step 808 and the reference image data. In other words, embodiments may provide for the use of histograms, edge features, short-time Fourier transforms, other algorithms, and combinations thereof in performing step 812. Depending on the algorithms and methods used in embodiments, step 812 may involve comparing colors, intensities, frequencies, bins, counts, contrast, exposure, dynamic range, etc.

Flow 800 passes from step 812 to decision 828 where a determination is made whether the image data captured at step 808 is similar, e.g., similar enough, to the reference image data compared at step 812. As noted above, in embodiments, the at least one processor that may perform step 812 may also make the determination at decision 828. The determination made at decision 828 may involve deciding that the image data captured at step 808 differs from reference data by less than some predetermined amount (e.g., some percentage). As noted above, the differences may relate to colors, intensities, frequencies, bins, counts, etc., depending on the specific algorithms and methods used.

The determination at decision 828 may in embodiments represent whether a disposable has been loaded correctly. For example, as illustrated in FIG. 6A and 6B, reference image data may reflect the image in FIG. 6A. In this embodiment, the image data taken at step 808 may be similar to image data of FIG. 6A (indicating a correctly loaded loop 500) or the image data may not be similar to the image data of FIG. 6A, e.g., image data of FIG. 6B (indicating an incorrectly loaded loop 500).

If at decision 828, a determination is made that the image data is not similar to the reference data, flow 800 passes to step 832 where an action is performed. In embodiments, step 832 may be performed to make an operator aware of an issue with the loaded disposable circuit, which if left uncorrected may cause a leak or other error during a separation process. In other embodiments, step 832 may be performed to prevent the separation process altogether until corrective action is performed.

In embodiments, step 832 may involve a number of sub-steps. For example, at sub-step 836 an audio alarm may be activated, e.g., a speaker or other audio device. At sub-step 840 a visual alarm may be activated, e.g., a light, a physical change, a change to a display screen (icon, message, flag, text, or other visual indicator).

In other embodiments, instead of, or in addition to an audio or visual alarm, a separation process may be stopped at step 844. In embodiments where a centrifuge is used to separate the composite liquid (e.g., system 100), rotation of the centrifuge may be stopped at optional step 848. As may be appreciated, in other embodiments, other components of a separation system may be stopped in order to stop the process at step 844.

In some embodiments, as part of performing an action at step 832, a number of sub-steps may be performed in combination. For example, in one embodiment, all of sub-steps 836, 840, 844, and 848 may be performed. In other embodiments, one or more combinations of sub-steps 836, 840, 844, and 848 may be performed. Flow 800 ends at 852.

Referring back to decision 828, if a determination is made that the image data is similar, in embodiments, flow 800 may end at 852. In these embodiments, flow 800 may be used to determine whether the disposable is initially loaded correctly.

In other embodiments, flow 800 may be used to continue to monitor the position of the disposable to determine whether it stays in a correct position throughout a separation process. In these embodiments, if a determination is made at decision 828 that the image data is similar, flow 800 passes to decision 856 where a determination is made as to whether the process, e.g., blood separation process, is over.

If at decision 856 a determination is made that the process is over, flow 800 ends at 852. If at decision 856 a determination is made that the process is not over, flow 800 may loop back and repeat steps 808, 812, and decision 828. The loop may continue until the process is over, which as noted above is determined at decision 856 and flow 800 then ends at 852.

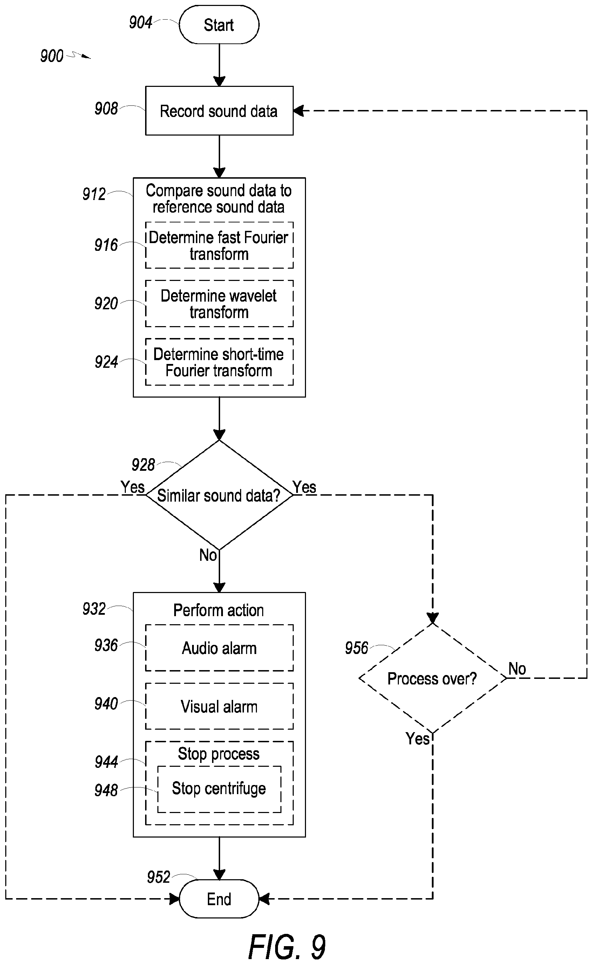

Referring now to FIG. 9, flow chart 900 starts at 904, and passes to step 908 where sound data is recorded. In embodiments, the sound data may be recorded by one or more microphones. In some embodiments, the one or more microphones that may be involved in performing step 908 may also be utilized for different functions. For example, in some embodiments, the microphones utilized to record sound data at step 908, may also be used to ensure other components of the separation system are operating properly. In other words, step 908 may be performed by portions of an audio system designed to perform a number of functions within the separation system.

From step 908, flow 900 passes to step 912, where the audio data recorded at step 908 is compared to reference sound data. The reference sound data may have been previously recorded and stored for comparison during performance of flow 900. In embodiments, step 912 may be performed, at least in part, by one or more processors. The at least one processor may in embodiments be part of a computer system such as system 1100 in FIG. 11 described below.

In some embodiments, the at least one processor may be a central processor that performs a number of other functions. In other embodiments, the at least one processor may be associated with a microphone and be located near the location of the microphone. Accordingly, the at least one processor may in embodiments be configured to specifically perform step 912, any of the optional steps 916, 920, and 924, as well as determination 928 (described further below).

As shown in FIG. 9, step 912 may involve a number of sub-steps. For example, the comparison may involve determining a fast Fourier transform of the sound data at optional step 916. The fast Fourier transform determined at optional step 916 may be compared with fast Fourier transform of the first reference sound data.

Similarly, step 912 may involve optional step 920 where a wavelet transform of the sound data captured at step 908 is determined. Step 912 may involve comparing the wavelet transform of the sound data captured at step 908 with the wavelet transform of reference sound data. In yet another embodiment, optional step 924 may be performed to determine a short-time Fourier transform of the sound data captured at step 908. The short-time Fourier transform of the sound data captured at step 908 may be compared with short-time Fourier transform of reference sound data.

In other embodiments, more than one of the optional steps 916, 920, and/or 924 may be performed during step 912. For example, in some embodiments, a combination of algorithms/methods may be used to make more accurate comparisons of the sound data captured at step 908 and the reference sound data. In other words, embodiments may provide for the use of wavelet transforms, fast Fourier transforms, short-time Fourier transforms, other algorithms, and combinations thereof in performing step 912. Depending on the algorithms and methods used in embodiments, step 912 may involve comparing intensities, frequencies, counts, etc.

Flow 900 passes from step 912 to decision 928 where a determination is made whether the sound data recorded at step 908 is similar, e.g., similar enough, to the reference sound data compared at step 912. As noted above, in embodiments, the at least one processor that may perform step 912 may also make the determination at decision 928. The determination made at decision 928 may involve deciding that the sound data captured at step 908 differs from reference sound data by less than some predetermined amount (e.g., some percentage). As noted above, the differences may relate to intensities, frequencies, counts, etc., depending on the specific algorithms and methods used.

The determination at decision 928 may in embodiments represent whether a disposable has been loaded correctly. For example, when a disposable is loaded correctly into a centrifuge assembly, e.g., a loop positioned in the correct bearing locations (FIG. 6A), the centrifuge when spun may create a particular sound. If for example the disposable is not loaded correctly (FIGS. 5A and 5B), the sound created when the centrifuge is spun may be different.

If at decision 928, a determination is made that the sound data is not similar to the reference data (e.g., a disposable loaded incorrectly), flow 900 passes to step 932 where an action is performed. In embodiments, step 932 may be performed to make an operator aware of an issue with the loaded disposable circuit, which if left uncorrected may cause a leak or other error during a separation process. In other embodiments, step 932 may be performed to prevent the separation process altogether until corrective action is performed.

Step 932 may involve a number of sub-steps in embodiments. For example, at sub-step 936 an audio alarm may be activated, e.g., a speaker or other audio device. At sub-step 940 a visual alarm may be activated, e.g., a light, a physical change, a change to a display screen (icon, message, flag, text, or other visual indicator).

In other embodiments, instead of, or in addition to an audio or visual alarm, a separation process may be stopped at step 944. In embodiments where a centrifuge is used to separate the composite liquid (e.g., system 100), rotation of the centrifuge may be stopped at optional step 948. As may be appreciated, in other embodiments, other components of a separation system may be stopped in order to stop the process at step 944.

In some embodiments, as part of performing an action at step 932, a number of sub-steps may be performed in combination. For example, in one embodiment, all of sub-steps 936, 940, 944, and 948 may be performed. In other embodiments, one or more combinations of sub-steps 936, 940, 944, and 948 may be performed. Flow 900 ends at 952.

Referring back to decision 928, if a determination is made that the sound data is similar, in embodiments, flow 900 may end at 952. In these embodiments, flow 900 may be used to determine whether the disposable is initially loaded correctly.

In other embodiments, flow 900 may be used to continue to monitor whether a disposable remains in a correct position throughout a separation process. In these embodiments, if a determination is made at decision 928 that the sound data is similar, flow 900 passes to decision 956 where a determination is made as to whether the process, e.g., blood separation process, is over.

If at decision 956 a determination is made that the process is over flow 900 ends at 952. If at decision 956 a determination is made that the process is not over, flow 900 may loop back and repeat steps 908, 912, and decision 928. The loop may continue until the process is over, which as noted above is determined at decision 956 and flow 800 then ends at 952.

Flow chart 1000 starts at 1004, and passes to step 1008 where a light source is activated. In embodiments, the light source may be directed to a particular location of a separation system. For example, the light source may be directed to an edge of a centrifuge assembly, adjacent a channel where a separation vessel (e.g., from a disposable) may be positioned for separation of a fluid into components.

From step 1008, flow 800 passes to step 1012, where scattered light from the light source may be detected. In embodiments, the scattering of light from the light source (activated at step 1008) may be indicative of an incorrectly loaded disposable. For example, as noted above with respect to FIGS. 4A and 4B, a portion of a vessel not fully positioned in a channel may scatter light that may be detected at step 1012.

Embodiments may provide for one or more sub-steps to be performed as part of step 1012. In some embodiments, step 1012 may be performed by a light detector, which may simply detect light at a location that when a disposable is loaded correctly does not receive light.

In other embodiments, image data from a location(s) may be captured. The image data may be of a position where a disposable is engageable with a portion of the separation system, e.g., a separation vessel within a channel (e.g., FIGS. 4A and 4B).

In these embodiments, image data may be captured by one or more cameras and compared to reference image data at optional step 1016. The reference image data may have been previously captured and stored for comparison during optional step 1016 and may correspond to a correctly loaded disposable. Optional step 1016 may be performed, at least in part, by one or more processors. The at least one processor may in embodiments be part of a computer system such as system 1100 in FIG. 11 described below.

Optional step 1016 may involve a number of optional sub-steps. For example, comparison of image data may involve determining a histogram of image data at optional step 1020. The histogram may be compared with a histogram of reference data.

In other embodiments, optional step 1016 may involve determining edge features at optional step 1024. The edge features of image data may be compared with edge features of reference data. In yet another embodiment, optional step 1028 may be performed to determine a short-time Fourier transform of image data. The short-time Fourier transform of the image data may be compared with short-time Fourier transform of reference data.

In other embodiments, more than one of the optional steps 1016, 1020, 1024, and/or 1028 may be performed in performing optional step 1016. For example, in some embodiments, a combination of algorithms/methods may be used to make more accurate comparisons of image data. In other words, embodiments may provide for the use of histograms, edge features, short-time Fourier transforms, other algorithms, and combinations thereof in performing step 1016. Depending on the algorithms and methods used in embodiments, step 1016 may involve comparing colors, intensities, frequencies, bins, counts, contrast, exposure, dynamic range, etc.

Flow 1000 passes from step 1012 to decision 1032 where a determination is made whether light has been detected at step 1012. In embodiments, the at least one processor that may perform step 1012 may also make the determination at decision 1032. The determination made at decision 1032 may in embodiments involve deciding that image data differs from reference data by less than some predetermined amount (e.g., some percentage). As noted above, the differences may relate to colors, intensities, frequencies, bins, counts, etc., depending on the specific algorithms and methods used.

The determination at decision 1032 may in embodiments represent whether a disposable has been loaded correctly. If at decision 1032, a determination is made that light has been detected (in embodiments indicating an incorrectly loaded disposable), flow 1000 passes to step 1036 where an action is performed. In embodiments, step 1036 may be performed to make an operator aware of an issue with the loaded disposable, e.g., a separation vessel, which if left uncorrected may cause a leak or other error during a separation process. In other embodiments, step 1036 may be performed to prevent the separation process altogether until corrective action is performed.

In embodiments, step 1036 may involve a number of sub-steps. For example, at sub-step 1040 an audio alarm may be activated, e.g., a speaker or other audio device. At sub-step 1044 a visual alarm may be activated, e.g., a light, a physical change, a change to a display screen (icon, message, flag, text, or other visual indicator).

In other embodiments, instead of, or in addition to an audio or visual alarm, a separation process may be stopped at step 1048. In embodiments where a centrifuge is used to separate the composite liquid (e.g., system 100), rotation of the centrifuge may be stopped at optional step 1052. As may be appreciated, in other embodiments, other components of a separation system may be stopped in order to stop the process at step 1048.

In some embodiments, as part of performing an action at step 1036, a number of sub-steps may be performed in combination. For example, in one embodiment, all of sub-steps 1040, 1044, 1048, and 1052 may be performed. In other embodiments, one or more combinations of sub-steps 1040, 1044, 1048, and/or 1052 may be performed. Flow 1000 ends at 1056.

Referring back to decision 1032, if a determination is made that no light was detected, in embodiments, flow 1000 may end at 1056. In these embodiments, flow 1000 may be used to determine whether the disposable is initially loaded correctly.

In other embodiments, flow 1000 may be used to continue to monitor the position of the disposable to determine whether it stays in a correct position throughout a separation process. In these embodiments, if a determination is made at decision 1032 that light is not detected, flow 1000 passes to decision 1060 where a determination is made as to whether the process, e.g., blood separation process, is over.

If at decision 1060 a determination is made that the process is over, flow 1000 ends at 1056. If at decision 1060 a determination is made that the process is not over, flow 1000 may loop back and repeat step 1012 and decision 1032. The loop may continue until the process is over, which as noted above is determined at decision 1060 and flow 1000 then ends at 1056.

Although flow charts 800, 900, and 1000 have been described with steps listed in a particular order, the embodiments are not limited thereto. In other embodiments, steps may be performed in different order, in parallel, or any different number of times, e.g., before and after another step. Also, flow charts 800, 900, and 1000 may include some optional steps or sub-steps. However, those steps above that are not indicated as optional should not be considered as essential to the invention, but may be performed in some embodiments of the present invention and not in others.

Also, flows 800, 900, and 1000 may be performed any number of times in sequence or parallel by different devices or by the same devices. For example, flow 900 may be performed in a first location where a disposable is engageable with a portion of a centrifuge assembly (e.g., a first bearing) and also at the same time (or in sequence) in a second location where the disposable is engageable with a portion of the centrifuge assembly (e.g., a second bearing). This is merely one example and other embodiments may provide for different variations, including performing each of flows 800 and 900 in parallel or in some sequence.

FIG. 11 illustrates example components of a basic computer system 1100 upon which embodiments of the present invention may be implemented. For example, separation device 104 (FIG. 1) as well as processor 428 (FIG. 3) may incorporate features of the basic computer system 1100 shown in FIG. 10. Computer system 1100 includes output device(s) 1004, and input device(s) 1008. Output device(s) 1104 include, among other things, one or more displays, including CRT, LCD, and/or plasma displays. Output device(s) 1104 may also include printers, speakers etc. Input device(s) 1108 may include a keyboard, touch input devices, a mouse, voice input device, scanners, etc.

Basic computer system 1100 may also include a processing unit 1112 and memory 1116, according to embodiments of the present invention. The processing unit 1112 may be a general purpose processor operable to execute processor executable instructions stored in memory 1116. Processing unit 1112 may include a single processor or multiple processors, according to embodiments. Further, in embodiments, each processor may be a single core or a multi-core processor, having one or more cores to read and execute separate instructions. The processors may include general purpose processors, application specific integrated circuits (ASICs), field programmable gate arrays (FPGAs), and other integrated circuits.

The memory 1116 may include any tangible storage medium for short-term or long-term storage of data and/or processor executable instructions. The memory 1116 may include, for example, Random Access Memory (RAM), Read-Only Memory (ROM), or Electrically Erasable Programmable Read-Only Memory (EEPROM). Other storage media may include, for example, CD-ROM, tape, digital versatile disks (DVD) or other optical storage, tape, magnetic disk storage, magnetic tape, other magnetic storage devices, etc.

Storage 1128 may be any long-term data storage device or component. Storage 1128 may include one or more of the devices described above with respect to memory 1116. Storage 1128 may be permanent or removable.

Computer system 1100 also includes communication devices 1136. Devices 1136 allow system 1100 to communicate over networks, e.g., wide area networks, local area networks, storage area networks, etc., and may include a number of devices such as modems, hubs, network interface cards, wireless network interface cards, routers, switches, bridges, gateways, wireless access points, etc.

The components of computer system 1100 are shown in FIG. 11 as connected by system bus 1140. It is noted, however, that in other embodiments, the components of system 1100 may be connected using more than a single bus.

It will be apparent to those skilled in the art that various modifications and variations can be made to the methods and structure of the present invention without departing from its scope. Thus, it should be understood that the invention is not limited to the specific examples given.

While example embodiments and applications of the present invention have been illustrated and described, it is to be understood that the invention is not limited to the precise configuration and resources described above. Various modifications, changes, and variations apparent to those skilled in the art may be made in the arrangement, operation, and details of the methods and systems of the present invention disclosed herein, which scope is defined by the appended claims.

## Claims

1. A system (400) for identifying incorrect loading of a disposable in a fluid separation system, the system comprising:
a microphone (424) for recording sound from the fluid separation system, wherein the microphone is configured to record the sound from an incorrectly loaded disposable; and
a processor (428) connected to the microphone and configured to, after the microphone records the sound from the incorrectly loaded disposable, send a signal to perform an action;
wherein the processor is configured to compare the recorded sound to first reference sound data, wherein when the recorded sound differs from the first reference sound data, the at least one processor sends a signal to perform a notification action; and
the comparing utilizes one or more of a fast Fourier transform, a wavelet transform, a short-time Fourier transform, and combinations thereof.

2. The system of claim 1, wherein the action comprises an alarm.

3. The system of claim 1 or claim 2, wherein the action comprises an audio alarm.

4. The system of any one of claims 1-3, wherein the action comprises a visual alarm.

5. The system of any one of claims 1-4, wherein after the microphone records the sound from the incorrectly loaded disposable, the processor sends a signal to stop rotation of a centrifuge of the fluid separation system.

6. A method of identifying incorrect loading of a disposable in a fluid separation system, the method comprising:
recording first sound data of a centrifuge assembly that is loaded with a disposable; and
comparing, by at least one processor (428), the first sound data to first reference sound data,
when the first sound data differs from the first reference sound data, the at least one processor sending a signal to perform a notification action;
wherein the comparing utilizes one or more of a fast Fourier transform, a wavelet transform, a short-time Fourier transform, and combinations thereof.

7. The method of claim 6, wherein the comparing comprises determining a first fast Fourier transform of the first sound data and comparing the first fast Fourier transform to a second fast Fourier transform of the first reference sound data.

8. The method of claim 6 or 7, wherein when the first sound data differs from the first reference sound data, the at least one processor sends a signal to stop a rotation centrifuge of the centrifuge assembly.

## Patentansprüche

1. System (400) zum Identifizieren einer falschen Einsetzung eines Einwegartikels in einem Fluidtrennsystem, wobei das System Folgendes umfasst:
ein Mikrofon (424) zum Aufzeichnen von Ton aus dem Fluidtrennsystem, wobei das Mikrofon konfiguriert ist, um den Ton von einem falsch eingesetzten Einwegartikel aufzuzeichnen; und
einen Prozessor (428), der mit dem Mikrofon verbunden ist und konfiguriert ist, um, nachdem das Mikrofon den Ton von dem falsch eingesetzten Einwegartikel aufgezeichnet hat, ein Signal zu senden, um eine Aktion durchzuführen;
wobei der Prozessor konfiguriert ist, um den aufgezeichneten Ton mit ersten Referenztondaten zu vergleichen, wobei, wenn sich der aufgezeichnete Ton von den ersten Referenztondaten unterscheidet, der mindestens eine Prozessor ein Signal sendet, um eine Benachrichtigungsaktion durchzuführen; und
das Vergleichen eine oder mehrere von einer schnellen Fourier-Transformation, einer Wavelet-Transformation, einer Kurzzeit-Fourier-Transformation und Kombinationen davon verwendet.

2. System nach Anspruch 1, wobei die Aktion einen Alarm umfasst.

3. System nach Anspruch 1 oder Anspruch 2, wobei die Aktion einen akustischen Alarm umfasst.

4. System nach einem der Ansprüche 1 bis 3, wobei die Aktion einen visuellen Alarm umfasst.

5. System nach einem der Ansprüche 1 bis 4, wobei der Prozessor, nachdem das Mikrofon den Ton von dem falsch eingesetzten Einwegartikel aufgezeichnet hat, ein Signal zum Anhalten einer Rotation einer Zentrifuge des Fluidtrennsystems sendet.

6. Verfahren zum Identifizieren einer falschen Einsetzung eines Einwegartikels in einem Fluidtrennsystem, wobei das Verfahren Folgendes umfasst:
Aufzeichnen erster Tondaten einer Zentrifugenanordnung, in die ein Einwegartikel eingesetzt ist; und
Vergleichen der ersten Tondaten mit ersten Referenztondaten durch mindestens einen Prozessor (428),
wenn sich die ersten Tondaten von den ersten Referenztondaten unterscheiden, Senden eines Signals durch den mindestens einen Prozessor, um eine Benachrichtigungsaktion durchzuführen;
wobei der Vergleich eine oder mehrere von einer schnellen Fourier-Transformation, einer Wavelet-Transformation, einer Kurzzeit-Fourier-Transformation und Kombinationen davon verwendet.

7. Verfahren nach Anspruch 6, wobei der Vergleich ein Bestimmen einer ersten schnellen Fourier-Transformation der ersten Tondaten und einen Vergleich der ersten schnellen Fourier-Transformation mit einer zweiten schnellen Fourier-Transformation der ersten Referenztondaten umfasst.

8. Verfahren nach Anspruch 6 oder 7, wobei der mindestens eine Prozessor, wenn sich die ersten Tondaten von den ersten Referenztondaten unterscheiden, ein Signal zum Anhalten einer Rotationszentrifuge der Zentrifugenanordnung sendet.

## Revendications

1. Système (400) pour identifier le chargement incorrect d'un élément jetable dans un système de séparation de fluide, le système comprenant :
un microphone (424) pour enregistrer le son du système de séparation de fluide, dans lequel le microphone est configuré pour enregistrer le son d'un élément jetable chargé incorrectement ; et
un processeur (428) connecté au microphone et configuré pour, après que le microphone a enregistré le son de l'élément jetable chargé incorrectement, envoyer un signal pour effectuer une action ;
dans lequel le processeur est configuré pour comparer le son enregistré à des premières données sonores de référence, dans lequel lorsque le son enregistré diffère des premières données sonores de référence, l'au moins un processeur envoie un signal pour effectuer une action de notification; et
la comparaison utilise une ou plusieurs parmi une transformée de Fourier rapide, une transformée en ondelettes, une transformée de Fourier à court terme et des combinaisons de celles-ci.

2. Système selon la revendication 1, dans lequel l'action comprend une alarme.

3. Système selon la revendication 1 ou la revendication 2, dans lequel l'action comprend une alarme sonore.

4. Système selon l'une quelconque des revendications 1 à 3, dans lequel l'action comprend une alarme visuelle.

5. Système selon l'une quelconque des revendications 1 à 4, dans lequel, après que le microphone a enregistré le son de l'élément jetable chargé incorrectement, le processeur envoie un signal pour arrêter la rotation d'une centrifugeuse du système de séparation de fluide.

6. Procédé d'identification d'un chargement incorrect d'un élément jetable dans un système de séparation de fluide, le procédé comprenant :
l'enregistrement de premières données sonores d'un ensemble de centrifugeuse qui est chargé d'un élément jetable ; et
la comparaison, par au moins un processeur (428), des premières données sonores aux premières données sonores de référence,
lorsque les premières données sonores diffèrent des premières données sonores de référence, l'envoi par l'au moins un processeur d'un signal pour effectuer une action de notification ;
dans lequel la comparaison utilise une ou plusieurs parmi une transformée de Fourier rapide, une transformée en ondelettes, une transformée de Fourier à court terme et des combinaisons de celles-ci.

7. Procédé selon la revendication 6, dans lequel la comparaison comprend la détermination d'une première transformée de Fourier rapide des premières données sonores et la comparaison de la première transformée de Fourier rapide à une seconde transformée de Fourier rapide des premières données sonores de référence.

8. Procédé selon la revendication 6 ou 7, dans lequel lorsque les premières données sonores diffèrent des premières données sonores de référence, l'au moins un processeur envoie un signal pour arrêter une centrifugeuse en rotation de l'ensemble de centrifugeuse.
